# EUROPEAN PATENT APPLICATION

(11) **EP 1 264 892 A2**
(43) Date of publication of application: **11.12.2002**
(21) Application number: 02011588.7
(22) Date of filing: 27.05.2002
(51) Int. Cl.: C12N 15/867, C12N 15/63, C12N 15/90, A61K 48/00

(54) **Lentiviral vectors comprising endothelial cell specific promoters**

(30) Priority: 29.05.2001 IT MI20011138
(71) Applicant: Universita' Di Torino, 10124 Torino (IT)
(72) Inventor: Naldini, Luigi, 10134 Torino (IT); De Palma, Michele, 10098 Rivoli (IT)
(74) Representative: Modiano, Guido, Dr.-Ing.

(57) **Abstract**

A lentiviral vector construct for transferring nucleotide sequences *in vivo* and *ex vivo*, which comprises transcription regulatory sequences of one or more genes preferentially expressed in endothelial cells of mammals, particularly sequences of the intronic and promoter regions of one or more human and murine genes preferentially expressed in endothelial cells. The invention also relates to the use of a lentiviral vector construct according to the invention for manufacturing a preparation for treating an angiogenesis-related pathology by gene delivery and selective expression in cells engaged in angiogenesis.

## Description

The present invention relates to new transfer vectors, to a particle transducing said vectors, to methods for treating disorders involving angiogenesis that use said vectors, to the use of said vectors for manufacturing of medicaments for use in said treatment methods, and to methods for gene delivery and expression in cells engaged in angiogenesis by transducing said vectors *ex vivo*.

In particular, various methods are described for introducing the vector in cells engaged in angiogenesis and/or precursors thereof, both *in vivo* and *ex vivo,* in order to obtain selective gene expression *in vivo*.

Gene delivery *in vivo* is a powerful approach for studying biological processes and for developing innovative therapies. It is possible to achieve sustained and effective concentrations of proteins or RNA by *in situ* synthesis, in contrast with the technical difficulties of *in vitro* production and systemic administration.

Targeted delivery and expression of biologically active genes in the setting of pathological and tumor angiogenesis could be used to inhibit or promote the process and/or to interfere with other aspects of the underlying pathology. Targeted gene expression in cells involved in the neoformation of vessels may yield effective concentrations of a therapeutic biological factor only at the sites of the pathology, alleviating concerns about toxicity from direct administration of large doses and widespread expression in healthy tissues.

Tumor angiogenesis is a central process that controls tumor growth, is performed by genetically stable tissues and follows patterns that are common among heterogeneous tumors. Furthermore, it displays selective characteristics that are useful for specific targeting.

A tumor originates from the interaction of a heterogeneous population of transformed cells with the host tissues, to allow the progressive growth of the tumor mass and its support by newly developed vessels and stroma. These interactions have the following important features:
1. Vascular recruitment becomes an obligate and rate-limiting factor of growth as soon as the tumor reaches a critical mass and/or becomes invasive (1).
2. The population of tumor cells is heterogeneous and genetically unstable (2, 3). The vessels and stroma of the tumor are made of homogeneous and genetically stable tissues.
3. Tumors differ greatly for the origin of the cell types involved, according to the combination of genetic lesions driving their growth. Tumor angiogenesis must follow recurrent patterns.
4. Angiogenesis occurs only in few physiological conditions in adult individuals. Moreover, tumor angiogenesis may have distinctive features with respect to those observed in other conditions, such as wound healing (4-6).

Accordingly, angiogenic vessels and perivascular stromal cells are a homogeneous, stable and selective target shared among heterogeneous tumors, and cancer therapies directed against the recruitment of cells involved in vessel formation would be highly effective, broadly applicable, relatively non-toxic and poorly inducive to resistance.

However, the origin of the cells contributing to the formation of tumor vessels and their patterns of growth within the tumor are still obscure. Sequestration of preexisting vessels, sprouting of new branches and *in situ* vasculogenesis from bone marrow-derived precursors have been proposed as mechanisms contributing to different extents (7). Few conclusive data have been reported, partly due to the lack of experimental techniques allowing identification and selective marking of these cells *in vivo*.

The angiogenesis process might be the target of the delivery vehicle and of the activity of the therapeutic genes.

The gene transfer agents available until recently have had significant limitations for *in vivo* applications.

The aim of the present invention is to provide new gene transfer vectors that lead to the integration of genes efficiently in most tissue types but allows their selective expression only in cells engaged in angiogenesis.

An object of the present invention is to provide and express selectively genes in angiogenesis sites.

Another object of the present invention is to provide methods for the delivery of genes with anti-tumor activities, including but not limited to, suicide genes, immunomodulatory and proinflammatory cytokines, inhibitors of tissue invasion and of angiogenesis.

Another object of the present invention is to provide gene transfer vectors for targeting a disseminated metastatic disease, the most challenging and lethal condition of cancer.

Another object of the present invention is to provide gene transfer vectors and methods to allow biological interventions in all settings in which angiogenesis occurs. Examples include, but are not limited to, inhibiting retinal neovascularization and chronic inflammatory conditions characterized by angiogenesis, promotion of angiogenesis in ischemic tissues such as limbs or the myocardium.

Another object of the present invention is to provide gene transfer vectors for the delivery of a combination of pro-angiogenetic or anti-angiogenetic factors.

Another object of the present invention is to allow identification and screening of candidate target genes with biological activity in the various settings of angiogenesis mentioned above.

This aim, these objects and others that will become apparent hereinafter from the description that follows are achieved by lentiviral vector constructs for transferring nucleotide sequences *in vivo* and *ex vivo,* characterized in that it comprises transcription regulatory sequences of one or more genes preferentially expressed in endothelial cells of mammals.

The transcription regulatory sequences are preferably sequences from enhancers or promoter regions of one or more genes preferentially expressed in endothelial cells of mammals.

The genes preferentially expressed in endothelial cells are preferably human or murine, for example genes preferentially expressed in endothelial cells chosen from the group constituted by human or murine Tie2, Flk-1, Tie1, VE-Cad and ICAM-2.

In one embodiment, the lentiviral vector is derived from HIV-1.

In a preferred embodiment, the transcription regulatory sequences are sequences from the intronic and promoter regions of at least murine Tie2.

In another preferred embodiment, the transcription regulatory sequences are sequences from the intronic and promoter regions of at least human Tie2.

An example of a construct according to the present invention is a construct that comprises the sequences pRRLsin.mTie2Enh.cPPT.mTie2Pr.eGFP.Wpre. The intronic enhancer sequence of the mTie2 gene is placed upstream to the promoter. Other possible examples are lentiviral vector constructs that comprise the intronic enhancer sequence of the mTie2 gene in another position, such as upstream to the transgene between the cPPT and the promoter, in the vector LTR or downstream to the transgene.

The present invention also relates to a lentiviral vector particle that transduces the transfer vector according to the present invention.

The particle according to the present invention can include various envelopes, such as for example the envelopes of the VSV, LCMV, MLV, FLV, GALV or RD114.

The present invention also relates to a method for gene delivery and selective expression in cells engaged in angiogenesis by injection of the above-described vectors into the tissue affected by a pathology. The pathology includes, but is not limited to, ischemia of a tissue, retinal neovascularization or a chronic inflammatory disease.

The present invention also related to a method of gene delivery and selective expression in cells engaged in the angiogenesis of a tumor by injecting the above described vectors into the tumor mass.

The gene delivery and selective expression in cells engaged in angiogenesis can be provided by injecting the above described vectors into the bloodstream via a peripheral vein for systemic administration or in the afferent vasculature of the tissue affected by a pathology.

The disorder to be treated with the method according to the present invention includes, but is not limited to, tissue ischemia, retinal neovascularization, and a chronic inflammatory disease.

The gene delivery and selective expression in cells engaged in tumor angiogenesis and in metastasis can be provided by injecting the above described vectors into the bloodstream via a peripheral vein or into the afferent vasculature of the tumor or of the region(s) affected by metastasis.

The present invention also relates to a method of gene delivery and selective expression in cells engaged in angiogenesis in a pathological condition by transducing the above described vectors into bone marrow-derived progenitor cells *ex vivo* followed by transplant into conditioned or unconditioned recipients. The pathological condition includes, but is not limited to, tissue ischemia, retinal neovascularization, and a chronic inflammatory disease.

The present invention also relates to a method of gene delivery and selective expression in cells engaged in the angiogenesis of a tumor and in metastasis by transduction of the above described vectors into bone marrow-derived progenitor cells *ex vivo* followed by transplantation into conditioned and unconditioned recipients.

The invention also relates to the use of a lentiviral vector construct according to the present invention for manufacturing a preparation for use in the treatment of an angiogenesis-related pathology such as tissue ischemia, retinal neovascularization, and chronic inflammatory diseases, by delivery and selective expression of a gene in cells engaged in angiogenesis.

The preparation can be for administration by injection in the tissue affected by said pathology or for administration by injection into the bloodstream via a peripheral vein for systemic administration or in afferent vasculature of the tissue affected by said pathology.

In particular, according to the present invention, the gene is delivered in blood mononuclear cells *ex vivo* and the gene is selectively expressed *in vivo* in mononuclear cells.

The mononuclear cells for the gene selective expression *in vivo* one a distinct population of bone marrow-derived mononuclear cells expressing the Tie2 angiopoietin receptor.

The invention also relates to the use of a lentiviral vector construct according to the invention for manufacturing a preparation for use in the treatment of a tumor by delivery and selective expression of a gene in cells engaged in the angiogenesis of a tumor.

The preparation can be for administration by injection into the tumor mass or for administration into the bloodstream via a peripheral vein or in the afferent vasculature of the tumor or of the regions affected by metastasis.

The discussion that follows focuses on the angiogenesis that occurs in the onset of a tumor and its metastases. Similar considerations and similar strategies for gene targeting also apply to angiogenesis occurring in other pathologies, including a tissue ischemia, chronic inflammatory conditions or retinal neovascularization.

The Applicant studied, as a working model of angiogenesis, tumor angiogenesis in a tumor transplantation model in nude mice.

One method that was used involved direct delivery of vectors into the tumor mass. Another method that was used involved administration of a vector into the bloodstream, via a peripheral vein for systemic delivery or into the afferent vasculature of the tumor.

Selective targeting of cells engaged in angiogenesis was achieved by exploiting the efficient infection of a vector pseudotyped by the envelope of the vesicular stomatitis virus (VSV) or other envelopes with wide tropism, and by restricting expression of the transgene to endothelial cells and other cell types involved in angiogenesis by incorporation of specific transcription control elements in the vectors.

A large panel of regulatory and promoter regions from genes specifically expressed in endothelial cells and/or induced upon angiogenesis was tested in different combinations (in lentiviral vector) in order to achieve specificity of transgene expression. Several unique features of the lentiviral vector facilitated this approach.

Another method that was used involved *ex vivo* transduction of bone marrow progenitor cells with endothelium-targeted vectors and transplantation into marrow-depleted or unconditioned recipients.

The results of the Applicant have shown that the bone marrow contributed substantially to tumor vasculature and stroma. Efficient transduction of hematopoietic stem cells by lentiviral vectors allowed identification of gene-marked progeny in the tumor stroma and vasculature.

In the *ex vivo* strategy, a distinct population of mononuclear cells born *in vivo* from bone marrow stem cells transduced *ex vivo* with endothelium-specific vectors homed to the sites of angiogenesis, such as the tumor and its metastases, acting as vehicles of the tested or therapeutic gene.

It is also possible to use regulated vectors in combination with targeted expression, so that the expression of a therapeutic gene is temporally regulated, protecting against untoward effects of the therapy.

The vector according to the present invention can be used to express a transcription regulator, such as for example a tetracycline-dependent activator or repressor, selectively in cells engaged in angiogenesis, and would be combined with another vector containing the regulated expression cassette for the test or therapeutic genes.

Alternatively, both the targeted expression cassette for the regulator and the responsive cassette for the test or therapeutic genes can be incorporated in a single vector construct. Regulated targeted vectors are crucial in the strategy of bone marrow transduction *ex vivo,* given its potential for long-term production of transduced cells *in vivo*.

The Applicant used a new gene transfer system based on lentiviruses (hybrid lentiviral vectors, LV), which overcome several limitations of known systems (8-11); LV infect a wide range of tissues, integrate efficiency into the chromatin independently of cell division, do not transfer any viral genes, can be directly administered *in vivo,* allow sustained expression of the transgene, and do not need to overcome preexisting viral immunity.

Previous work has also improved the biosafety of LV, alleviating concerns raised by the use of vectors derived from pathogenic viruses (12-15).

The Applicant has developed several strategies to target LV in tumor angiogenesis, as detailed in the examples.

Some strategies for targeting viruses to tumor angiogenesis involved direct administration of LV into the tumor or into the bloodstream. Targeting to endothelial cells can be attempted at the level of infection by selecting endothelium-specific or engineered viral envelopes. These approaches have yielded, until now, vectors with too low infectivity to be used *in vivo* (16).

Vectors developed for targeting endothelial cells at the level of infection with endothelium-specific or engineered viral envelopes, however, can be combined with some of the strategies described in the present application.

Here the Applicant exploited the efficient infection of a vector pseudotyped by the VSV envelope and the restricted expression of the transgene to cells engaged in angiogenesis by incorporation of specific transcription control elements in the vector.

The Applicant collected a large panel of putative and established regulator/promoter regions from genes specifically expressed in endothelial cells and/or induced upon angiogenesis. The Applicant introduced them in different combinations into LV and tested them for specificity of transgene expression *ex vivo* and *in vivo.* Several unique features of LV facilitate this approach:
1. Self-inactivating vectors previously developed by the Applicant delete all viral transcription control regions and drive transgene expression solely from exogenous sequences introduced into the vector (14).
2. The minimal content of viral sequences appears to shield LV from the genome surveillance mechanism, which often leads to shut-off of retroviral vectors.
3. LV can deliver efficiently relatively large gene fragments, including complex regulatory elements (17).

Other strategies involved *ex vivo* transduction of bone marrow hematopoietic stem cells (HSC) with endothelium-targeted vectors, followed by repopulation of partly myeloablated hosts. Recent data, including the Applicant's experiments, support the view that the bone marrow contributes to tumor vasculature (18). LV transduced HSC with high efficiency and without requiring prolonged *ex vivo* manipulation or cytokine treatment (19-21, 11). These favorable conditions allowed the Applicant to demonstrate a substantial contribution of gene marked cells from the bone marrow to the vasculature and the stroma of the tumor.

The experiments described above provided proof of the principle of targeted gene delivery to the tumor. They can be applied to the delivery of therapeutic genes because:
1. Gene-based delivery facilitates administration of protein and RNA factors by targeting the molecular signals controlling cancer growth angiogenesis and metastasis.
2. Effective concentrations of factors can be achieved and maintained by *in situ* synthesis, as opposed to the technical challenges of *in vitro* production and systemic administration.
3. Delivery can be optimized before the therapeutic gene is chosen, since the pharmacokinetics is dependent on the carrier.
4. Expression was restricted to cells specifically engaged in angiogenesis. Direct administration of vectors *in vivo* and ubiquitous expression may preclude the use of several anti-cancer genes because of systemic toxicity, damage to healthy tissues, presentation of the product to the immune system by means of transduced antigen-presenting cells and immunization against it, uncontrolled dosage escalation owing to the growth of transduced tumor cells. Targeting the tumor vessels should yield effective concentrations of a therapeutic biological factor right where invasive and metastatic growths take place. These therapeutic factors expressed *in vivo* include conditioned cytotoxic factors, angiostatin, endostatins, interleukins, antiangiogenetic factors, immune-modulating factors.

In the *ex vivo* strategy, a distinct population of mononuclear cells born *in vivo* from HSC transduced *ex vivo* with endothelium-specific expression cassettes home to the tumor and its metastatic sites, acting as vehicles of the tested or therapeutic gene.

### EXAMPLES

### Generation of a lentiviral vector with selective expression in cells engaged in angiogenesis

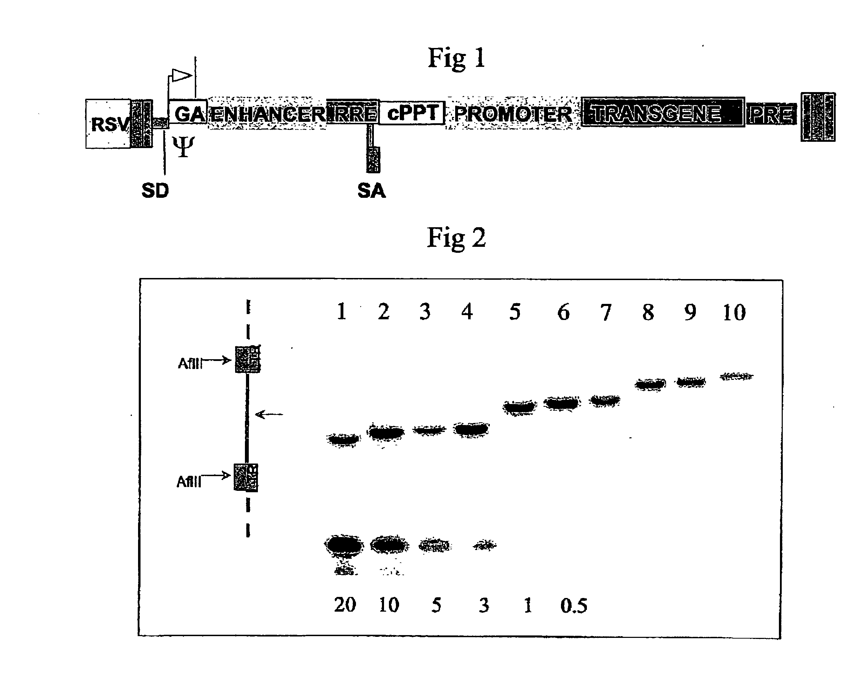

A schematic drawing of the integrated proviral vector, showing the major features of the lentiviral constructs, is shown in Figure 1.

Since transcription starting from the LTR is abolished due to a 400-nucleotide deletion in the U3 region (14), transcription of the eGFP reporter gene is under the control of an internal promoter and, in some cases, of additional regulatory sequences located in the 5' end of the vector, downstream of the Rev-response element and upstream of the splice acceptor sites.

*Cis*-acting sequences and post-transcriptional regulatory elements enhancing the expression of the transgene were also incorporated within the vector.

It has been shown that the insertion of a copy of the central polypurine tract (cPPT) upstream of the internal promoter significantly increased the efficiency of nuclear translocation of the vector genome in the host cell (11).

Furthermore, the element located at the 3' end of the genome of the woodchuck hepatitis virus (Wpre), reported to enhance export toward cytoplasm and/or polyadenylation of viral transcripts, was inserted at the 3' end of the vector, strongly increasing transcript levels in the cytoplasm of transduced cells.

A wide repertoire of putative endothelium-specific transcription regulatory elements was inserted upstream of the reporter eGFP gene in the lentiviral transfer construct. The following DNA clones were obtained through collaborations with Dr. U. Deutsch and Dr. G. Breier (Max-Planck-Institut, Bad Nauheim, FRG), and Prof. Bussolino (University of Torino):
1.8 kb fragment from the mouse Flk-1 promoter (22)
2.085 kb fragment containing the mouse Tie2 promoter (23)
510 bp fragment from the mouse Tie1 promoter
466 bp fragment from the human ICAM-2 promoter
2.5 kb fragment from mouse VE-cadherin promoter (24).
The following intronic enhancer sequences were also obtained:
1653 bp fragment from the Tie2 enhancer (23)
2.0 kb intronic enhancer from the mouse Flk-1 gene (25)
430 bp minimal enhancer from the mouse Flk-1 gene.

To generate pdmT2-short, a HindIII-HindIII 2084-bp fragment containing the murine Tie2 (mTie2) promoter (obtained from the pBSIIKS(+) - based derived from pHHSDKXK plasmid, kindly provided by Dr. U. Deutsch) was cloned into the lentiviral construct pRRLsin.hCMV.eGFP.Wpre in place of a ClaI-XbaI fragment corresponding to the human CMV (hCMV) promoter.

To generate a vector containing the cPPT sequence, the ClaI-KpnI 1880-bp fragment from the construct pRRLsin.cPPT.hCMV.eGFP.Wpre (11) was replaced with the BglII-KpnI 3356-bp fragment obtained from the vector containing the mTie2 promoter.

In the pdmT2-short construct (pRRLsin.cPPT.mTie2Pr.eGFP.Wpre), the reporter eGFP gene is under the transcriptional control of the murine Tie2 promoter.

To generate pdmT2, a XhoI-KpnI 1658-bp fragment, containing the mTie2 gene intronic enhancer obtained from pHHSDKXK, was inserted in sense or antisense orientation into the unique MfeI site present in the vector pmdHT2.

In addition to the mTie2 promoter, pdmT2 (pRRLsin.mTie2Enh.cPPT.mTie2Pr.eGFP.Wpre) contains regulatory elements present outside the 5' promoter region of the mTie2 gene and required for vascular-endothelial cell-specific expression (23; GenBank database accession no. U85629).

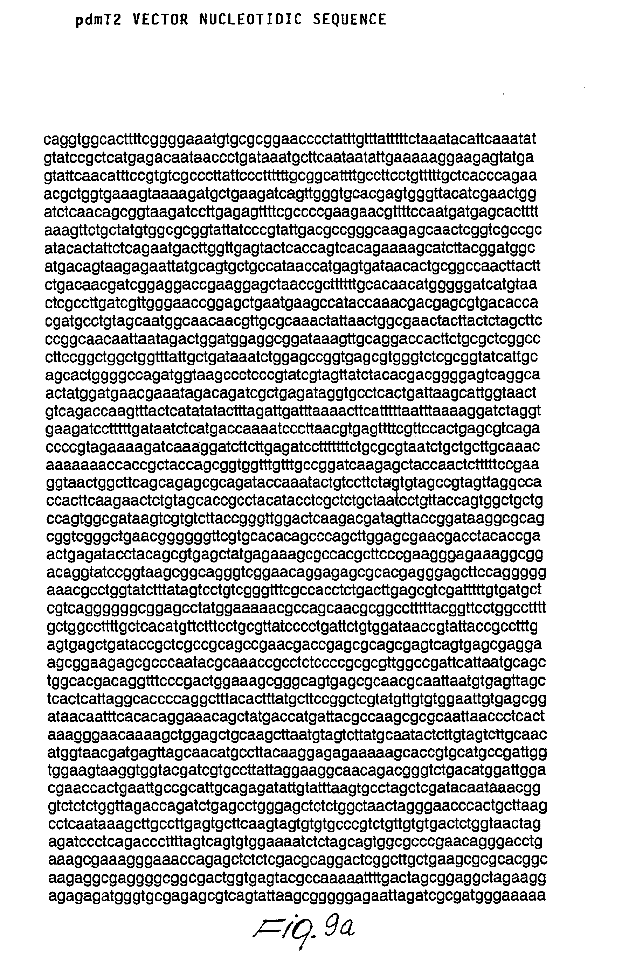

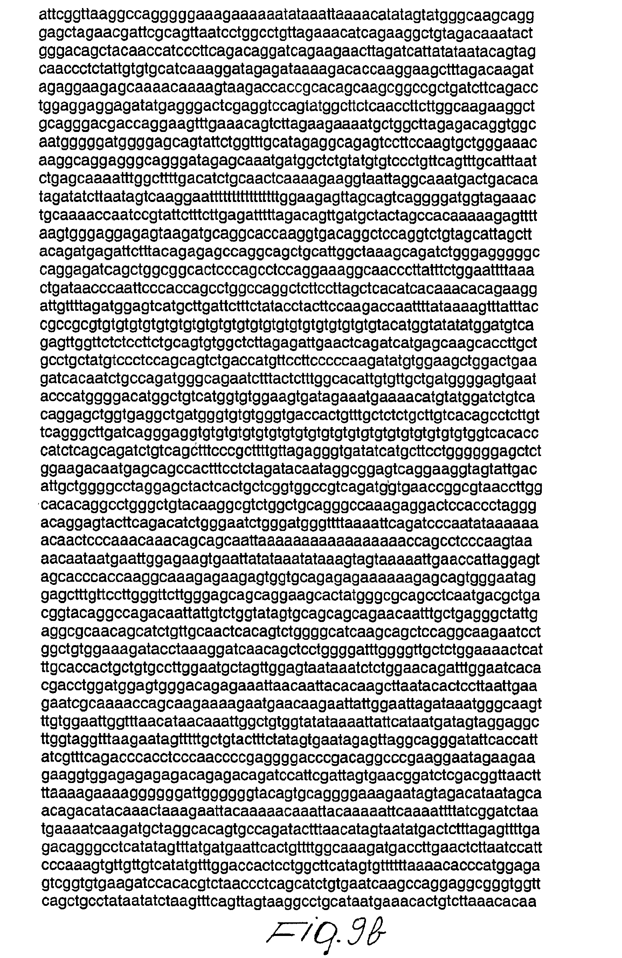

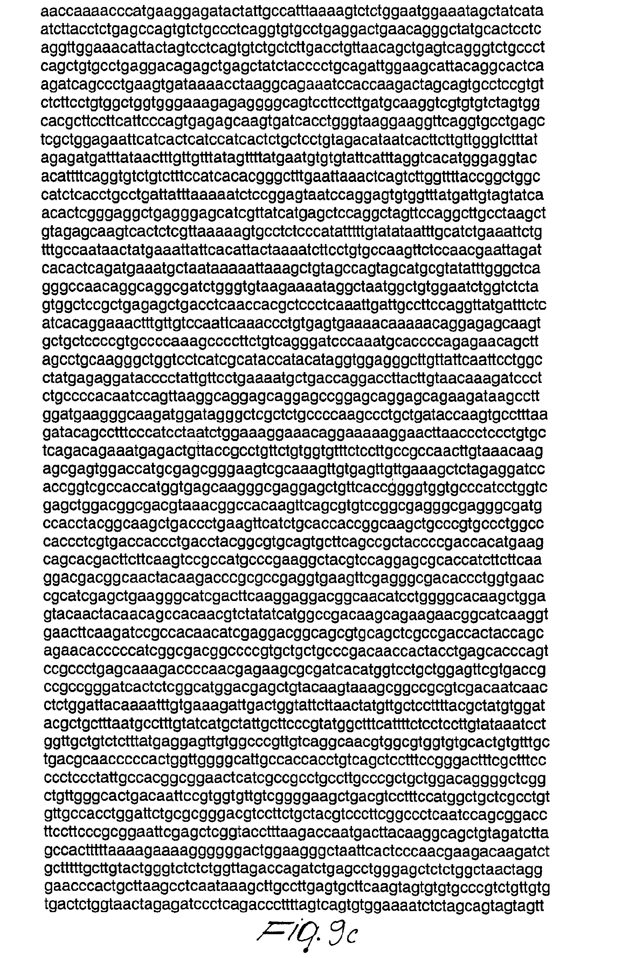

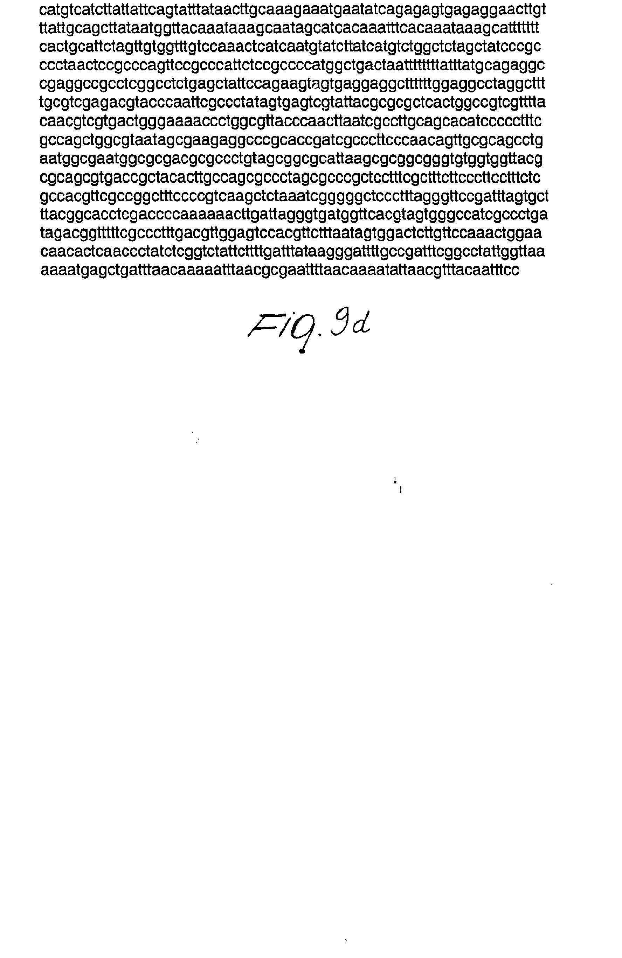

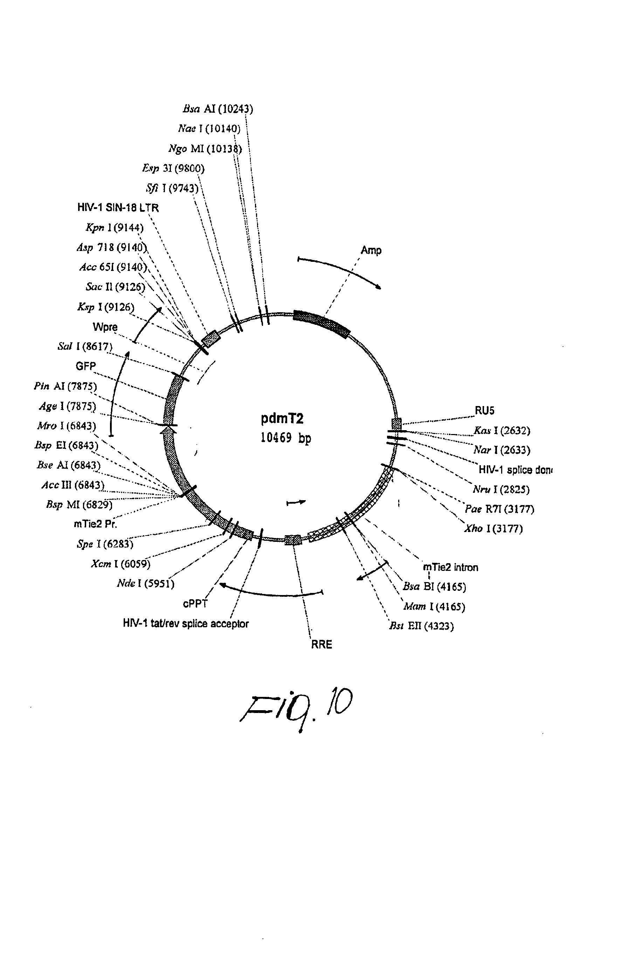

The nucleotide sequence and the map of the pdmT2 vector are shown in Figures 9 and 10.

### Validation of constructs in vitro

The efficiency of transfer of the expression cassette for each vector was evaluated by transduction of the HeLa cell line.

The conditioned medium of 293T cells used for vector production was concentrated 250X. Equal volumes of each vector stock were used to transduce HeLa cells.

Southern blot analysis of transduced cells confirmed the quality of the vector preparations on the basis of the comparable level of integration and stability of transgene expression.

Moreover, Southern blot analysis was also used to calculate the average number of integrated vector copies per genome of the transduced cells (Figure 2).

Genomic DNA purified from transduced HeLa cells (1. hICAM2 promoter; 2. mTie1 promoter; 3. hCMV promoter/enhancer; 4. hPGK promoter; 5. mFlk1 promoter; 6. mTie2 promoter; 7. mVE-Cad promoter; 8. mTie2 promoter/intronic enhancer, sense; 9. mTie2 promoter/intronic enhancer, antisense; 10. mFlk1 promoter/intronic enhancer), and a standard curve of plasmid DNA (20, 10, 5, 3, 1 and 0.5 equivalents of vector copies per genome) were digested with the restriction endonuclease AflII, which releases a vector fragment spanning from the 5'-LTR to the 3'-LTR.

A probe corresponding to the eGFP sequence was used to reveal the integrated vector. The number of integrated copies of vector per unit volume of stock was calculated to normalize the transduction activity of the various vector stocks in the following tests.

As a first experimental approach, a wide panel of immortalized cell lines and primary cultures was transduced *in vitro* with the entire repertoire of vectors. Among others, human and porcine primary endothelial cells, human primary fibroblasts, human primary lymphocytes, murine and human hematopoietic progenitors and their progeny, murine breast adenocarcinoma cells (TS/A) and the cell lines HeLa and 293T were used.

Each cell type was transduced with an amount of vector that produced stable integration and a comparable number of integrated copies for each vector.

Cytofluorimetric analysis of eGFP expression was performed at steady state transgene expression level, a condition which is achieved 5-7 days after transduction but depends mainly on the cell type, the transcriptional activity of the promoter, and the half-life of the transgene product.

Two variables were analyzed in order to select the most effective vector: i) the level of transgene expression in the target cell (endothelial type) had to be significant and had to be found in the majority of the cells, and ii) the level of transgene expression in the non-target cell (cells of non-endothelial type) had to be low or undetectable in the majority of cells. Taken together, these two parameters express the selectivity or specificity of expression of a given vector.

While the vectors carrying the ubiquitous h-CMV and h-PGK promoters drove eGFP expression to a very high level in all the cell types tested, most of the endothelial promoter vectors expressed the transgene at variable levels, but often in a non tissue-specific manner (data not shown).

However, cytofluorimetric analysis of transduced cells clearly indicated that the vectors containing regulatory sequences from the mTie2 gene fulfilled the required conditions better than the others, thus displaying the highest selectivity of expression for endothelial cells.

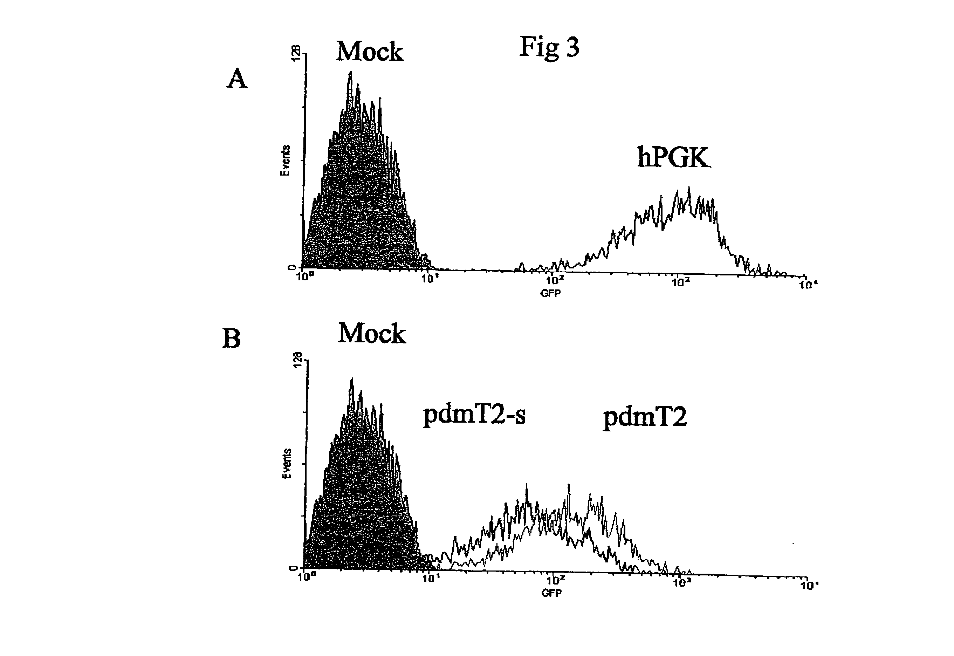

Selected data from these analyses are shown in Figures 2 and 3.

Figure 3 shows the transduction of primary human umbilical vascular endothelial cells (HUVEC). The histograms show the eGFP expression of mock-transduced cells (A and B, filled color), cells transduced with the ubiquitous hPGK vector (A, black line), with pdmT2-short (B, black line) and with pdmT2 (sense) (B, green line).

As shown in the figure, both vectors containing regulatory sequences from the mTie2 gene transduced and expressed the eGFP reporter gene in virtually all the cells in the tested population, thus meeting the first requirement for selective expression in endothelial cells.

Moreover, the pdmT2 vector allowed higher transgene expression than pdmT2-short, indicating that the *cis*-acting regulatory elements present outside the 5' promoter region of the mTie2 gene improved vector performance at least twofold (mean of fluorescence intensity: hPGK vector, 1476; pdmT2-short, 121; pdmT2 (sense), 252).

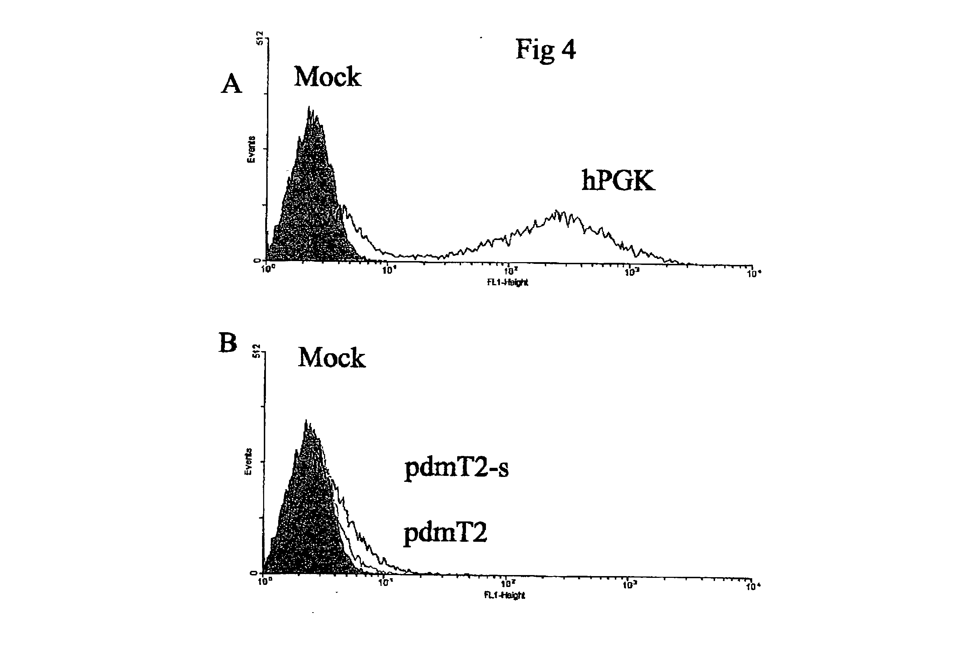

Figure 4 shows the transduction of murine breast adenocarcinoma cells (TS/A). The histograms show the eGFP expression of mock-transduced cells (A and B, filled color), cells transduced with the ubiquitous hPGK vector (A, black line), with pdmT2-short (B, black line) and with pdmT2 (sense) (B, green line).

Expression driven by the pdmT2-short and pdmT2 (sense) vectors is almost undetectable and in any case occurs only in less than 2% of the total cell population (mean of fluorescence intensity: 25-30), whereas the hPGK vector expressed the transgene in 72% of the total population (mean of fluorescence intensity: 524).

These results clearly indicate that transgene expression is strongly impaired in the tested non-endothelial cell line, thus meeting the second requirement for selective expression in endothelial cells.

Comparable results were obtained when testing other non-endothelial cell types (data not shown).

Moreover, the pdmT2 vector determined a lower transgene expression than pdmT2-short, indicating that the intronic enhancer of the mTie2 gene present in the latter vector improved specificity of expression by repressing transgene expression in non-endothelial cells. Comparable results were obtained when testing other non-endothelial cell types (data not shown).

To confirm the transgene expression data, Northern blot analysis was performed with total RNA purified from 293T cells transduced with the entire repertoire of vectors. Transcripts containing the eGFP sequence were almost undetectable in samples obtained from 293T cells transduced with pdmT2-short and pdmT2, whereas low to high levels of RNA expression were found in total RNA samples from cells transduced with the other endothelial cell-specific or ubiquitous vectors (data not shown).

In conclusion, pdmT2 was found to be the most effective vector for endothelial targeting because of the low basal level of transgene expression in non-endothelial cells and because of the high level obtained in endothelial cells *in vitro.*

The Applicant then assessed the significance of the basal levels of transgene expression detected by sensitive cytofluorimetric analysis, by delivery of a conditionally toxic gene to permissive (endothelial, HUVEC) and non-permissive (non-endothelial, 293T) cells, using the pdmT2 vectors and the vector containing the hCMV promoter.

The eGFP sequence was replaced with a bicistronic expression cassette for the bacterial nitroreductase gene, followed by an IRES-eGFP sequence.

The ratio between resistance and killing of transduced cells upon treatment with the pro-drug metronidazole was evaluated. Treatment with metronidazole did not induce cell death in 293T cells transduced with the pdmT2 vector, although these cells expressed low but detectable levels of the eGFP gene.

In contrast, toxicity was clearly observed in 293T cells transduced with the control hCMV vector and in HUVEC cells transduced with the pdmT2 vectors and with the vector containing the hCMV promoter (data not shown).

These results indicated that the basal levels of transgene expression driven by the pdmT2 vector in non-endothelial cells had a limited biological significance.

### Systemic delivery of pdmT2

The abovementioned experiments defined pdmT2 as the best candidate vector for endothelial cell targeting.

To further validate the endothelial specificity of pdmT2, an *in vivo* model of tumor angiogenesis was devised. Specifically, the tumorigenic cell line TS/A, derived from a murine breast adenocarcinoma, was used to induce subcutaneous tumors in immunocompromised mice.

The TS/A tumor in the host animal recruits endothelial cells efficiently via angiogenesis/vasculogenesis. These cells are identified well by immunostaining with the vessel marker PECAM-1 (CD31).

Different strategies of vector delivery were exploited to assess the transduction efficiency and tissue-specificity of pdmT2, in comparison with vectors containing the ubiquitously expressed hPGK and hCMV promoters.

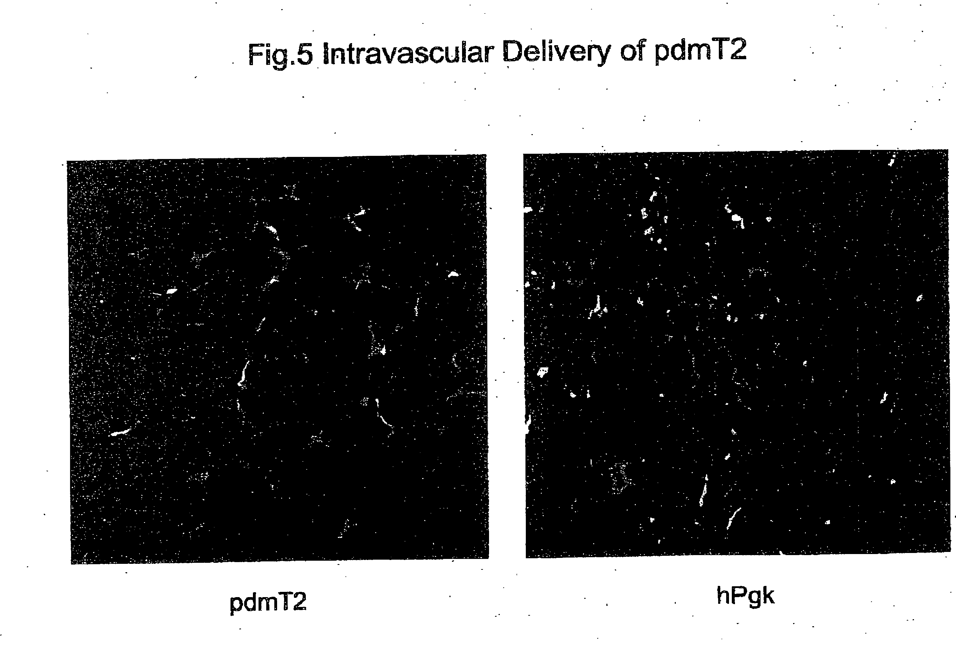

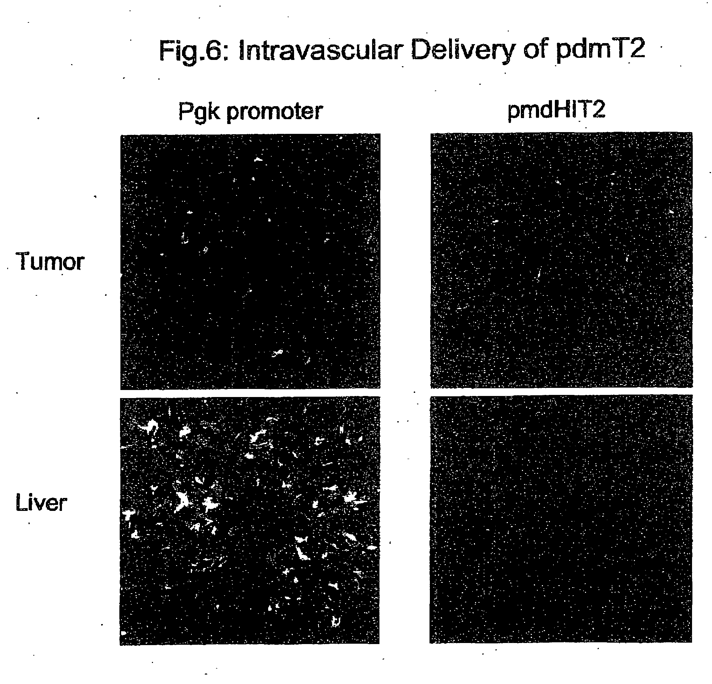

The first delivery approach tested was the intravenous administration of the vector. 5*10^5 TS/A cells were injected subcutaneously in nude mice. One week later, when the tumors had reached a diameter of 4-5 mm, a vector dose corresponding to 1-5*10^8 infectious particles was injected into the tail vein of the mice. One week after administration of pdmT2, immunohistochemical analysis on tumor sections revealed well-defined co-localization of eGFP and of the endothelial cell markers PECAM-1 (Figure 5) and VE-cadherin, whereas almost no eGFP positive cells could be detected in other well-vascularized organs, including the liver (Figure 6), the lungs and the heart.

By contrast, the control vector carrying the PGK promoter had determined a widespread and high level of eGFP expression in the tumor mass (Figure 5) and in other organs, such as the liver (Figure 6).

The data shown indicated that pdmT2 transcription data was limited to a fraction of the endothelial cells that constitute the vessels of the tumor, since no other cell type present in the tissue was positive for eGFP expression.

Furthermore, eGFP expression was undetectable in endothelial cells constituting the vessels of the other organs analyzed. Taken together, these results indicated that when intravenous delivery was performed, transgene expression from the new vector was specifically observed in the endothelial cells engaged in the tumor angiogenetic process.

### Intratumoral delivery of pdmT2 vector

A second experimental approach for *in vivo* validation of pdmT2 was intratumoral delivery of the vector. Escalating doses (1-10*10^7 infectious particles) of vector were injected within the tumor mass, both at early stages, when the tumor was merely visible, and later, when it had reached a larger size.

The tumors were dissected and analyzed at increasingly longer times after injection in order to monitor the appearance and destiny of transduced cells *in vivo*.

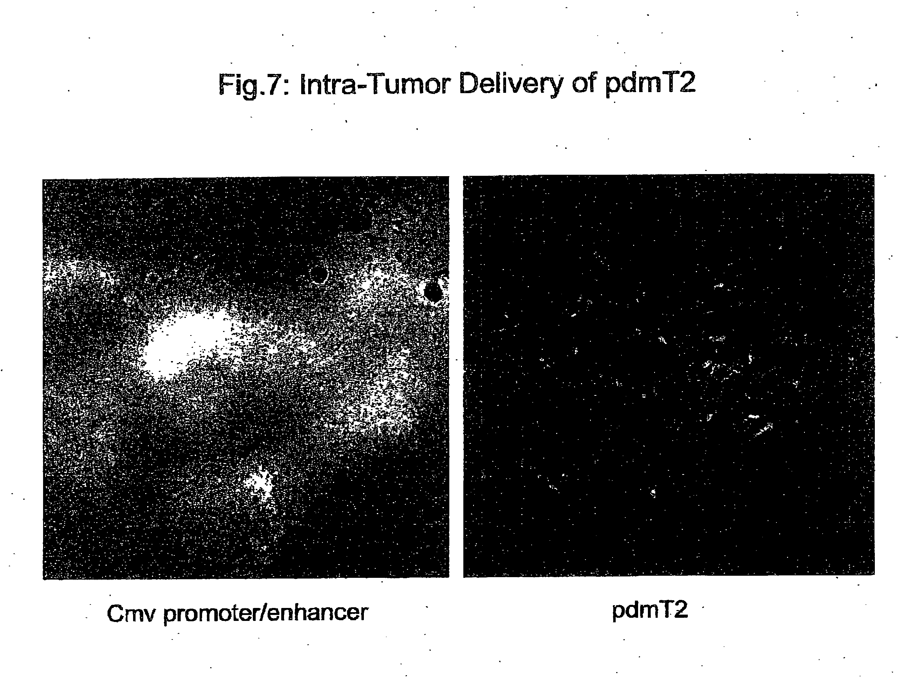

In these experiments, the control vector carrying the hCMV promoter transduced the tumor mass to a very high extent (Figure 7). At variance, pdmT2-driven eGFP expression was observed only in a more dispersed cell population (Figure 7) located mainly at the periphery of the tumor, which expressed vascular and hematopoietic markers, such as PECAM-1 (CD31), CD45 and Sca1 (data not shown).

These stromal cells, expressing the reporter eGFP gene under the control of the regulatory sequences of the mTie2 gene, are suspected to be involved in the angiogenetic process that leads to tumor vascularization.

This hypothesis was suggested by the following data: i) most of the eGFP positive cells were found to be incorporated into, or in close association with, the small blood vessels of the tumor; ii) the eGFP positive cells did not express any granulocyte or lymphoid differentiation markers and did not co-distribute with common tumor-infiltrating monocytes/macrophages, which represent a significant proportion of stromal population; iii) the cellular Tie2 receptor gene was demonstrated previously to be over-expressed in endothelial cells engaged in angiogenesis.

Despite the different transduction pattern obtained by intratumoral delivery with respect to intravenous delivery, the above mentioned results indicated an efficient and selective transduction of endothelial cells and of non-endothelial cells likely to be involved in angiogenesis.

### Ex vivo transduction of bone marrow cells by pdmT2 vector and in vivo transplantation

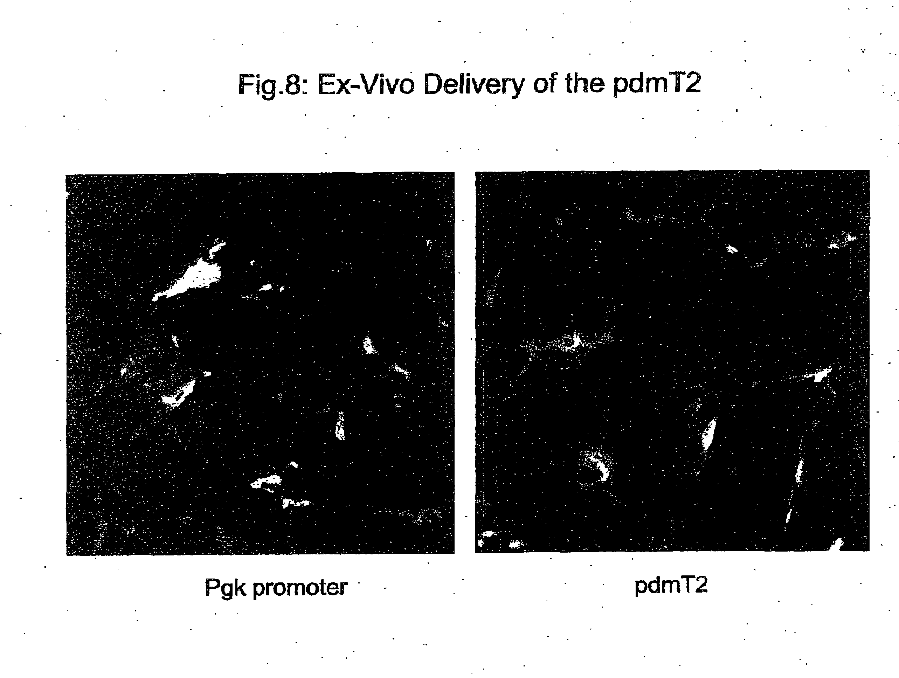

To investigate the origin of the cells transduced by the pdmT2 vector, hematopoietic stem cells purified from mouse bone marrow were transduced with pdmT2 and with the vector containing the hPGK promoter and were transplanted into irradiated nude mice. Two months after the transplant, tumor cells were injected subcutaneously and the tumor was examined two weeks later (Figure 8).

Immunohistochemical analysis of tumor sections revealed that the bone marrow-derived cells contributed predominantly to the formation of tumor stroma. Most of the cells expressing the eGFP transgene under the hPGK promoter were found to be of hematopoietic lineage (CD45 positive), mainly co-localizing with monocyte-macrophage differentiation markers (CD11b, F480 positive) (Figure 8).

By contrast, when the pdmT2 vector was used, transgene expression was restricted to a subpopulation of mononuclear stromal cells with distinctive features such as the distribution pattern within the tumor mass, their frequency and the expression profile of surface markers. The targeted cells were negative for the expression of granulocyte and lymphoid markers while expressed different combinations of vascular/hematopoietic markers, such as PECAM-1 (CD31), CD45 and CD11b (not shown) (Figure 8).

As obtained with intratumoral delivery of pdmT2, but here with a higher degree of specificity, most of these cells became incorporated in a vascular network at the tumor periphery where tissue invasion and angiogenesis take place or found associated with or incorporated into small blood vessels throughout the tumor mass. These cells expressed the myeloid marker CD11b but could be distinguished: i) from common monocyte/macrophage lineage cells by the specific recruitment to the tumor, the selective distribution at its periphery and the expression of other surface markers such as Tie2, Sca1 and CD34 (low); ii) from tumor-infiltrating neutrophils, by the morphology and surface markers, including the lack of expression of the granulocyte marker Gr-1. From the outer to the inner periphery of the tumor, the GFP-positive cells became incorporated in a network of tubular or laminar structures displaying specific marking patterns such as homogeneous expression of Tie2, Sca1 and CD34 and spotted, complementary expression of CD11b (with GFP) and CD31, suggesting the alternative occurrence of BM-derived non EC and tissue-derived EC. These results indicate that the pdmT2 vector targets a distinct population of mononuclear cells that originate from the bone marrow-derived, are capable of repopulating the host and are recruited efficiently into the sites of angiogenesis in the tumor. Only a fraction of these cells were identified as true endothelial cells by CD31 expression and typical morphology.
We suggest that the subset of bone marrow-derived cells that we describe here and that were targeted specifically by the pdmT2 vector (TEM, Tie2 Expressing Mononuclear cells) may have been previously scored among EC and macrophages at the periphery of tumors and be specifically responsible for some of the previously reported vascular growth-promoting activities. While the functions of these newly identified cells remain to be ascertained, our results provide the means to purify, characterize them and also to track their route to the tumor. We found that a small fraction of blood mononuclear cells expressed the pdmT2 vector and distinguishing surface features similar to those of the GFP+ cells in the tumors of transplanted mice. Preliminary results suggest that these circulating cells contribute to the tumor TEM cell population. Selective transgene expression in these cells *in vivo* can allow targeting therapies in the primary tumor and in its metastatic sites.

The results have proved the principle of effective gene transfer and of selective expression in cells engaged in tumor angiogenesis.

Clarifying these processes and identifying the involved molecular elements are crucial tasks for the advancement of the understanding of cancer and for validating potential targets of new therapies. By transferring targeted genes on cells engaged in the angiogenesis of a tumor, the Applicants have identified their tissue source and their manner of growth in the tumor, achieving proof of effective gene transfer and of selective expression in cells engaged in angiogenesis in the tumor.

The disclosures in Italian Patent Application No. MI2001A001138 from which this application claims priority are incorporated herein by reference.

## Claims

1. A lentiviral vector construct for transferring nucleotide sequences *in vivo* and *ex vivo*, **characterized in that** it comprises transcription regulatory sequences of one or more genes preferentially expressed in endothelial cells of mammals.

2. The construct according to claim 1, **characterized in that** said transcription regulatory sequences are sequences from the enhancer and promoter regions of one or more genes preferentially expressed in endothelial cells of mammals.

3. The construct according to claim 1 or 2, **characterized in that** said genes preferentially expressed in endothelial cells are human or murine.

4. The construct according to any one of the preceding claims, **characterized in that** said lentiviral vector is derived from HIV-1.

5. The construct according to any one of the preceding claims, **characterized in that** said genes preferentially expressed in endothelial cells are selected from the group consisting of human or murine Tie2, Flk-1, Tie1, VE-Cad and ICAM-2.

6. The construct according to any one of the preceding claims, **characterized in that** it comprises transcription regulatory sequences from the intronic enhancer and promoter regions at least of murine Tie2.

7. The construct according to any one of claims 1 to 5, **characterized in that** it comprises transcription regulatory sequences from the intronic and promoter regions at least of human Tie2.

8. The construct according to claim 1, comprising transcription regulatory sequences from the intronic enhancer of at least one of murine Tie2 and human Tie2.

9. The vector construct according to claims 1 to 8, **characterized in that** said vector is a targeted regulated expression vector.

10. The construct according to any one of claims 1 to 6, **characterized in that** it comprises the sequences pRRLsin.mTie2Enh.cPPT.mTie2Pr.eGFP.Wpre arranged in this order or with the intronic enhancer sequence of the mTie2 gene located in a different position upstream to the promoter, in the vector LTR or downstream to the transgene.

11. A lentiviral vector particle that transduces the transfer vector according to any one of claims 1 to 10.

12. The lentiviral vector particle according to claim 11, **characterized in that** it comprises a VSV glycoprotein envelope.

13. The lentiviral vector particle according to claim 11, **characterized in that** it comprises a murine leukemia amphotropic virus envelope.

14. Use of a lentiviral vector construct according to any one of claims 1 to 10 for manufacturing a preparation for treating an angiogenesis-related pathology by delivery and selective expression of a gene in cells engaged in angiogenesis.

15. The use according to claim 14, **characterized in that** said pathology is selected from the group consisting of tissue ischemia, retinal neovascularization, and chronic inflammatory disorders.

16. The use according to one of claims 14 or 15, **characterized in that** said preparation is for administration by injection into the tissue affected by said disorder.

17. The use according to one of claims 14 or 15, **characterized in that** said preparation is for administration by injection into the bloodstream via a peripheral vein for systemic administration or into afferent vasculature of the tissue affected by said disorder.

18. Use of a lentiviral vector construct according to one of claims 1 to 10 to produce a preparation for treating a tumor by delivery and selective expression of a gene in cells engaged in tumor angiogenesis.

19. The use according to claim 18, **characterized in that** said preparation is for administration by injection into the tumor mass.

20. The use according to claim 18, **characterized in that** said preparation is for administration into the bloodstream via a peripheral vein or into the afferent vasculature of the tumor or of the regions affected by metastasis.

21. The use according to any one of claims 14-20, **characterized in that** said preparation comprises a lentiviral vector particle according to any one of claims 11-13.

22. A method of gene delivery and selective expression in cells responsible for angiogenesis in a pathological condition, comprising transduction of a transfer vector according to any one of claims 1 to 10 in marrow-derived progenitor cells *ex vivo*.

23. The method according to claim 22, **characterized in that** said pathology is selected from the group consisting of tissue ischemia, retinal neovascularization and chronic inflammatory disorders.

24. A method of gene delivery and selective expression in cells responsible for angiogenesis in a tumor and metastasis, comprising transduction of a transfer vector according to any one of claims 1 to 10 in marrow-derived progenitor cells *ex vivo.*
